## Europäisches Patentamt
## European Patent Office
## Office européen des brevets

(19)

(11) Numéro de publication: **0 178 228**
**B1**

(12)

# FASCICULE DE BREVET EUROPÉEN

(45) Date de publication du fascicule du brevet:
02.08.89

(51) Int. Cl.⁴: **A 61 L 9/12**

(21) Numéro de dépôt: **85401953.6**

(22) Date de dépôt: **07.10.85**

(54) **Diffuseur de liquides volatils.**

(30) Priorité: **09.10.84 FR 8415473**

(43) Date de publication de la demande:
**16.04.86 Bulletin 86/16**

(45) Mention de la délivrance du brevet:
**02.08.89 Bulletin 89/31**

(84) Etats contractants désignés:
**AT BE CH DE FR IT LI LU NL SE**

(56) Documents cité:
**EP-A-0 028 852**

(73) Titulaire: **Reckitt & Colman S.A., 15, rue Ampère, F-91301 Massy Cédex (FR)**

(72) Inventeur: **Lhoste, Jean- François, 6 rue du Général Delestrain, F-28000 Chartres (FR)**
Inventeur: **Delage, Thierry, 65 rue Pierre Demours, F-75107 Paris (FR)**

(74) Mandataire: **Rinuy, Santarelli, 14, avenue de la Grande Armée, F-75017 Paris (FR)**

EP 0 178 228 B1

LIBER, STOCKHOLM 1989

## Description

La présente invention concerne un diffuseur de liquides volatils pour créer une ambiance, par exemple pour odoriser ou désodoriser l'atmosphère d'une pièce.

On connait un grand nombre de dispositifs destinés à cette fin. Ils comprennent pratiquement tous une mèche imprégnée du liquide volatil dont on désire répandre les vapeurs dans l'atmosphère, et des moyens permettant en cours d'utilisation de mettre ladite mèche au contact de l'air ambiant et pendant la période de non utilisation de protéger cette mèche en évitant son contact avec l'air.

De façon générale dans le cas d'une mèche plongeant partiellement dans un liquide contenu dans un flacon et pour mettre la partie de la mèche émergeant du liquide au contact de l'air il faut, soit extraire cette mèche du flacon qui la contient après en avoir ôté un capot d'obturation, soit dévisser plus ou moins un capot d'obturation du flacon pour dégager plus ou moins une coiffe de protection de ladite mèche tout en permettant à l'air de circuler entre ladite coiffe et ledit capot grâce à des lumières ou fentes ménagées dans ce dernier, soit encore mettre ladite mèche au contact direct d'une autre matière absorbante logée dans un capot d'obturation du flacon muni de fentes ou de lumières de circulation d'air après avoir ôté une coiffe de protection de ladite mèche.

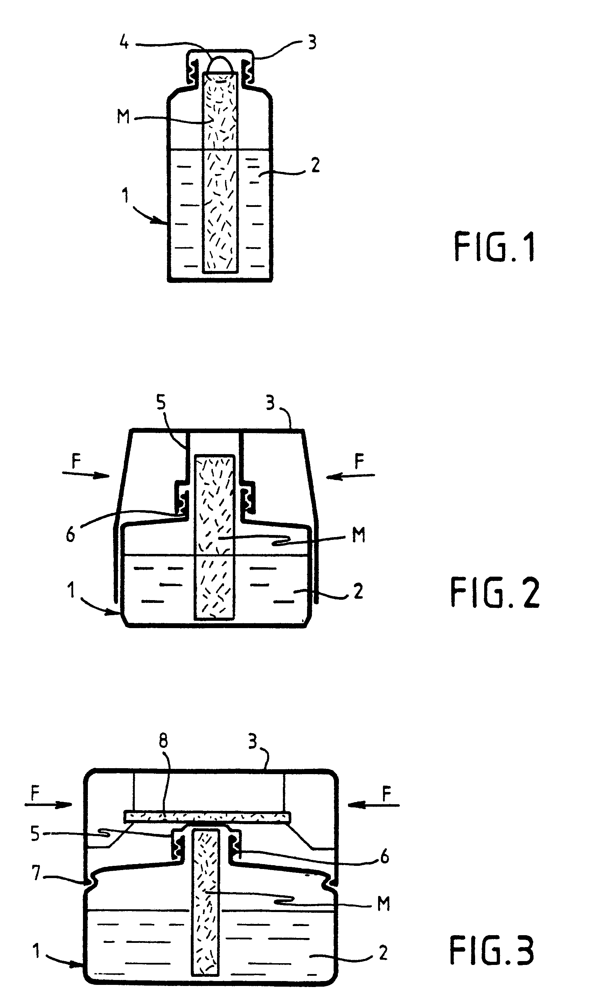

Ces différentes variantes sont illustrées très schématiquement dans leur principe sur les figures 1 à 3 annexées.

Ainsi, sur la figure 1 on a représenté un flacon 1 contenant un liquide volatil 2 dans lequel baigne partiellement une mèche M. Ce flacon est obturé par un capot ou bouchon obturateur 3. Pour obtenir une diffusion des vapeurs du liquide 2 dans l'atmosphère, il suffit de dévisser et d'ôter le bouchon 3, de tirer plus ou moins sur la mèche M hors du flacon au moyen de l'anneau 4. Pour arrêter la diffusion on procède aux opérations inverses. Outre ces manipulations, on constate que l'ensemble est inesthétique en position de fonctionnement et qu'il faut veiller à conserver le bouchon 3 sous peine de pertes inutiles de liquide lorsque la diffusion n'est pas nécessaire et l'impossibilité d'arrêter la diffusion ou de transporter sans risque de fuite, l'ensemble diffuseur, si ce bouchon 3 est égaré.

Sur la figure 2 on a représenté également un flacon 1 avec son liquide dans lequel baigne partiellement la mèche M. La partie supérieure de celle-ci est protégée par une coiffe 5 solidaire de la partie interne du capot ou bouchon obturateur 3. La jupe verticale de ce dernier se visse sur la face externe correspondante du flacon 1. La partie supérieure de cette jupe comporte en F des fentes ou fenêtres. Le col 6 du flacon est en prise, par filetage, avec la coiffe 5.

Pour obtenir la diffusion il suffit de dévisser le capot 3. L'air circule alors par l'espace dégagé du col 6 et par les fentes ou fenêtres F du capot 3 en entraînant les vapeurs du liquide dont est imprégnée la partie de la mèche M ainsi mise au contact de l'air. Là encore un problème d'esthétique se pose en ce sens qu'en dévissant le capot 3, on augmente la hauteur de l'ensemble donc la forme générale du dispositif.

Enfin, sur la figure 3 on a représenté un flacon 1 avec son liquide volatil 2 dans lequel baigne partiellement la mèche M. Le capot ou bouchon obturateur 3 est en prise avec le flacon 1, par exemple par encliquetage ou filetage en 7. La partie de la mèche M émergeant du col 6 du flacon 1 est protégée par une coiffe 5 se vissant sur ce col. Par ailleurs, l'intérieur du capot 3 est muni d'une plaque 8 de substance absorbante en tout matériau approprié tel que, par exemple, en mousse de cellulose et analogues. La disposition de cette plaque est telle que lorsque l'on retire la coiffe 5, cette plaque se trouve au contact de la partie supérieure de la mèche M. Ainsi lorsque l'on veut se servir de ce dispositif, on commence par retirer le capot 3; on dévisse et retire la coiffe 5 et on replace le capot en l'enfonçant suffisamment pour que la mèche M vienne au contact de la plaque 8. Pour arrêter la diffusion, on procède aux opérations inverses. Il faut évidemment veiller à conserver la coiffe 5 de manière à éviter toute perte inutile du produit à diffuser. Bien entendu, la jupe du capot 3 est munie en F de fentes ou fenêtres permettant la circulation de l'air. Bien entendu, en cas de perte de ladite coiffe 5, il est impossible d'arrêter totalement la diffusion et de transporter l'ensemble sans risque de fuite de liquide.

La présente invention obvie aux inconvénients signalés ci-dessus, touchant aussi bien au domaine de l'esthétique qu'au domaine pratique. Elle vise un diffuseur à mèche présentant la caractéristique de réaliser une étanchéité évitant une diffusion inutile du produit en position "fermée" de régler la diffusion du liquide volatil par variation du débit d'air le traversant, et de la surface de diffusion exposée au flux d'air tout en gardant au dispositif lui-même sa forme initiale,

Le diffuseur selon l'invention est essentiellement constitué d'un flacon dans lequel se trouve le liquide à diffuser, liquide dans lequel baigne partiellement une mèche; d'un capot ou bouchon obturateur monté sur ledit flacon par encliquetage tout en ayant la possibilité de tourner autour de son axe sans translation, ledit capot étant muni de fentes ou fenêtres pour le passage de l'air d'un support de mèche encliqueté sur le col dudit flacon et bloqué en rotation, ce support étant en forme de manchon muni de fentes ou de fenêtres pour le passage de l'air et d'un filetage extérieur; d'un bouchon comprenant d'une part, une jupe filetée intérieurement pour être en prise avec le filetage extérieur dudit support de mèche et comportant au surplus des stries verticales extérieures destinées à venir en prise avec un manchon vertical solidaire de la partie supérieure dudit capot, et, d'autre part, un manchon intérieur formant coiffe d'étanchéité pour ladite mèche.

D'autres caractéristiques et les avantages de l'invention ressortiront plus clairement de la description qui va suivre faite en regard des figures 4 à 7 annexées sur lesquelles:

- la figure 4 est une vue en élévation en coupe d'un diffuseur selon l'invention en position dite "d'arrêt";
- la figure 5 est une vue en perspective d'un détail de la figure 4;
- la figure 6 est une vue en perspective d'un autre détail de la figure 4; et
- la figure 7 est une vue correspondant à la figure 4, le diffuseur étant en position dite de "marche".

En se référant à ces figures, le diffuseur selon l'invention comporte un flacon 1 dans lequel se trouve le liquide volatil 2 dans lequel baigne partiellement la mèche M. La partie émergeante supérieure de cette mèche est protégée comme on le verra ci-après, par un système qui sera ultérieurement décrit. La partie supérieure du flacon 1 est constituée par un capot ou obturateur 3 de forme appropriée encliquetée en 3a sur le flacon 1 et dont la périphérie est munie de fentes ou fenêtres, dites d'aération, F. La partie supérieure de ce capot est munie intérieurement d'un manchon 9, comportant des stries verticales destinées à venir en prise avec la périphérie extérieure d'un bouchon 10 lequel, par son filetage intérieur 15 vient lui-même en prise avec le filetage extérieur 16 d'un support de mèche 11 comportant en sa périphérie des fentes ou fenêtres V. Une jupe intérieure 12 du bouchon 10 forme coiffe pour la partie supérieure émergeante de la mèche M. Cette dernière est à son tour supportée et maintenue en position verticale par une pièce cylindrique 13 à bords évasés venant s'encliqueter sur le col 6 du flacon 1 d'une part, et rendue solidaire, d'autre part, de la pièce 11.

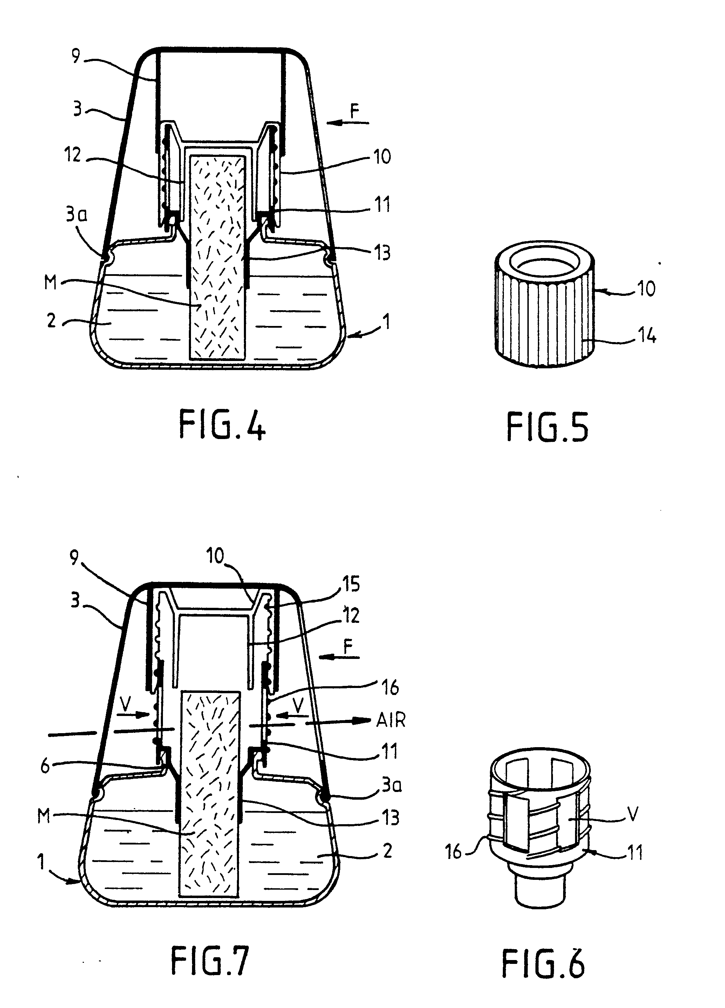

Lorsque le diffuseur n'est pas utilisé, le bouchon 10 est, comme représenté à la figure 4, à sa position la plus basse. Il obture les fentes V du support de mèche 11 et sa jupe 12 coiffe la partie supérieure de la mèche M. Aucune aération n'étant possible, le liquide 2 ne peut se dégager dans l'atmosphère. Il y a étanchéité parfaite. Lorsqu'il s'agit de diffuser les vapeurs dudit liquide volatil, il suffit d'imprimer au capot 3 un mouvement de rotation. Cette rotation se traduit alors par l'entraînement, au moyen du manchon 9 et par les stries 14 prévues sur la paroi extérieure du bouchon 10, par la rotation dudit bouchon 10, lequel effectue alors un mouvement de translation vers le haut. Il s'ensuit que la remontée du bouchon 10 dégage les fentes ou fenêtres V du support de mèche 11 ce qui permet à l'air de circuler aussi bien par les fentes ou fenêtres F du capot que par les fentes ou fenêtres V du support de mèche. L'ouverture de ces fentes ou fenêtres est ainsi réglable en fonction de la hauteur à laquelle le bouchon 10 est amené, cette hauteur dépendant du nombre de tours que l'on a imprimé au capot 3.

La rotation de ce capot n'ayant modifié en rien l'aspect général du dispositif, l'esthétique de ce dernier est preservée, le réglage du débit d'air en circulation dans l'appareil se faisant sans intervenir sur la position relative du flacon et du capot obturateur.

Le dispositif selon l'invention permet, en le fermant, d'arrêter totalement la diffusion à tout instant, en réalisant ainsi un ensemble étanche favorable au transport, sans démontage et sans avoir besoin de remettre en place une pièce d'obturation.

Bien entendu, comme dans tout diffuseur, le liquide volatil peut être du type parfum, matière ou substance antiseptique ou assainissante.

De même, la mèche M peut être en tout matériau assurant une diffusion par phénomène de capillarité. On citera par exemple les matériaux du type fibres, papier, cellulose, feutre, acétate de cellulose, polyester, polypropylène.

## Revendication

Diffuseur de liquides volatils pour créer une ambiance, pour odoriser ou désodoriser une pièce, diffuseur essentiellement caractérisé par le fait qu'il est constitué d'un flacon (1) dans lequel se trouve le liquide à diffuser (2), liquide dans lequel baigne partiellement une mèche (M); d'un capot obturateur (3) monté sur ledit flacon par encliquetage (3a) tout en ayant la possibilité de tourner autour de son axe sans translation verticale, ledit capot étant muni de fentes ou fenêtres (F) pour le passage de l'air; d'un support de mèche (11-13) encliqueté sur le col (6) dudit flacon et bloqué en rotation, ce support étant en forme de manchon muni de fentes ou fenêtres (V) pour le passage de l'air et d'un filetage extérieur; d'un bouchon interne (10) comprenant d'une part une jupe filetée intérieurement (15) pour être en prise avec le filetage extérieur (16) dudit support de mèche et comportant au surplus des stries verticales extérieures (14) destinées à venir en prise avec un manchon vertical (9) solidaire de la partie supérieure dudit capot (3) et, d'autre part, un manchon intérieur (12) formant coiffe d'étanchéite pour ladite mèche.

## Patentanspruch

Verdunstungseinrichtung für flüchtige Flüssigkeiten zur Erzeugung einer Umgebung zum Odorisieren oder Desodorisieren eines Raumes, welche Verdunstungseinrichtung im wesentlichen dadurch gekennzeichnet ist, daß sie aus einer Flasche (1), in der sich die abzugebende Flüssigkeit (2) befindet, in die ein Docht (M) teilweise eingetaucht ist; aus einer Verschlußkappe (3), die auf der Flasche durch Verrasten (3a) befestigt ist, dabei jedoch die Möglichkeit hat, sich um ihre Achse ohne vertikale Verschiebung zu drehen, wobei die Kappe mit Schlitzen oder Fenstern (F) zum Durchtritt von Luft versehen ist; aus einem Dochthalter (11-13), der am Hals (6) der Flasche verrastet und gegen Verdrehen gesichert ist, wobei der Halter die Form einer mit Schlitzen oder Fenstern (V) zum Durchtritt der Luft und mit einem Außengewinde versehenen Hülse hat; und aus einem inneren Verschluß (10) besteht, der einerseitse eine Schürze (15), die mit einen Innengewinde zum Eingriff mit dem Außengewinde (16) des Dochthalters versehen ist und darüber hinaus vertikale äußere Rillen (14) hat, die dazu bestimmt sind, mit einem mit dem oberen Teil der Kappe (3) fest verbundenen vertikalen Stutzen (9) in Eingriff zu gelangen, und andererseits eine innere Hülse (12) aufweist, die eine Abdichtkappe für den Docht bildet.

## Revendication

Volatile liquid diffuser for creating an ambiance, for odorising or de-odorising a room, essentially characterized in that it comprises a flask (1) containing the liquid (2) to be diffused in which is partly immersed a wick (M); a cap (3) snap-fastened (3a) to said flask and able to rotate about its axis without vertical movement in translation, said cap incorporating slots or windows (F) for air to pass through; a wick support (11-13) snap-fastened to the neck (6) of said flask and prevented from rotating, in the form of a sleeve incorporating slots or windows (V) for air to pass through and an external screwthread; an internal stopper (10) comprising on the one hand an internally screwthreaded skirt (15) adapted to engage the external screwthread (16) on said wick support and comprising external vertical striations (14) adapted to engage a vertical sleeve (9) fastened to the upper part of said cap (3) and on the other hand an internal sleeve (12) forming a sealed cap for said wick.

FIG.1

FIG.2

FIG.3

EP 0 178 228 B1

FIG.4

FIG.5

FIG.7

FIG.6

3